# EUROPEAN PATENT SPECIFICATION

(11) **EP 2 404 502 B1**
(45) Date of publication and mention of the grant of the patent: **10.04.2019**
(21) Application number: 11172876.2
(22) Date of filing: 06.07.2011
(51) Int. Cl.: A01N 37/42, A61K 8/49, A61Q 19/00, A01N 59/20, A01N 37/46, A61K 31/195, A01P 1/00, A01N 43/36

(54) **COMPOSITIONS CONTAINING PYRROLIDONE CARBOXYLIC ACID (PCA) AND METALLIC SALTS**
Zusammensetzungen enthaltend Pyrrolidon-carbonsäure und Metallsalze
Compositions contenant de l'acide pyrrolidone carboxylique et des sels métalliques

(30) Priority: 07.07.2010 IT MI20101252
(43) Date of publication of application: 11.01.2012
(73) Proprietor: Laboratori Baldacci S.P.A., 56124 Pisa (IT)
(72) Inventor: Baldacci, Massimo, I-56124 Pisa (IT)
(74) Representative: Pistolesi, Roberto

(56) References cited:
- WO-A1-2004/064867
- WO-A2-01/28552
- FR-A1- 2 938 193
- US-B1- 6 475 501
- CLAR E J ET AL: "L'acide pyrrolidone carboxylique (PCA) et la peau//Pyrrolidone carboxylic acid and the skin", INTERNATIONAL JOURNAL OF COSMETIC SCIENCE, KLUWER ACADEMIC PUBLISHERS, DORDRECHT, NL, vol. 3, no. 3, 1 January 1981 (1981-01-01) , pages 101-113, XP009097785, ISSN: 0142-5463, DOI: 10.1111/J.1467-2494.1981.TB00275.X

## Description

PCA, represented in the Formula 1 below, is a cyclic organic compound, more commonly known as pyrrolidone carboxylic acid or pyroglutamic acid.

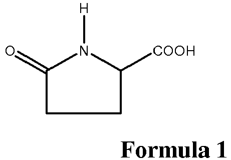

The carrier action thereof at promoting and improving the gastrointestinal absorption of some active ingredients is known in literature.

Furthermore, it should be considered, just like urea and lactic acid, a natural moisturizing factor (NMF).

Actually, due to the moisturizing and emollient properties thereof it is used as an ingredient in various cosmetic compositions especially as a moisturizer for the skin and hair balsam.

This compound is free of adverse effects when applied at skin level.

Recent publications describe the virucidal activity of PCA with respect to *Rhinovirus* after application on the hands *(*Journal of Antimicrobial Chemotheraphy 2005, 56, 805-807) besides the antibacterial action thereof.

Furthermore, both the antibacterial activity of copper against bacteria of the *Staphilococcus* and *Salmoin* strains and the antibacterial and immune system reinforcement activity of zinc are known from literature (Martindale, The complete Drug Reference, 33th Edition, 1999).

The adverse effects of such metals which, when applied on the skin or on the mucosae, may cause irritation and sensitiveness which may lead to actual allergies are also known.

Now, it has been surprisingly discovered that the association between PCA and at least one salt, preferably a copper, zinc or manganese salt, synergically potentiates the antiviral and antibacterial action of PCA.

### Brief description of the figures

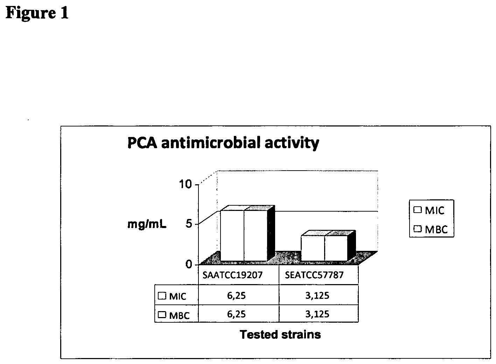

Figure 1: MIC and MBC of PCA on *Staphylococcus aureus* (SA) and *Staphylococcus epidermidis* (SE)

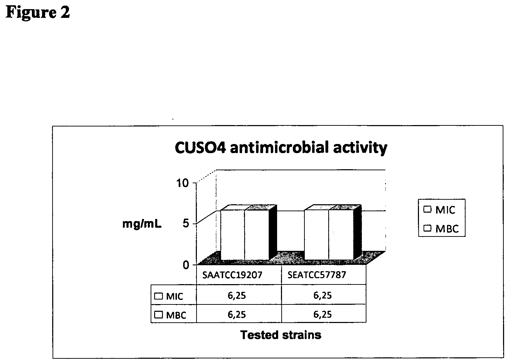

Figure 2: MIC and MBC of CuSO₄ on *Staphylococcus aureus* (SA) and *Staphylococcus epidermidis* (SE)

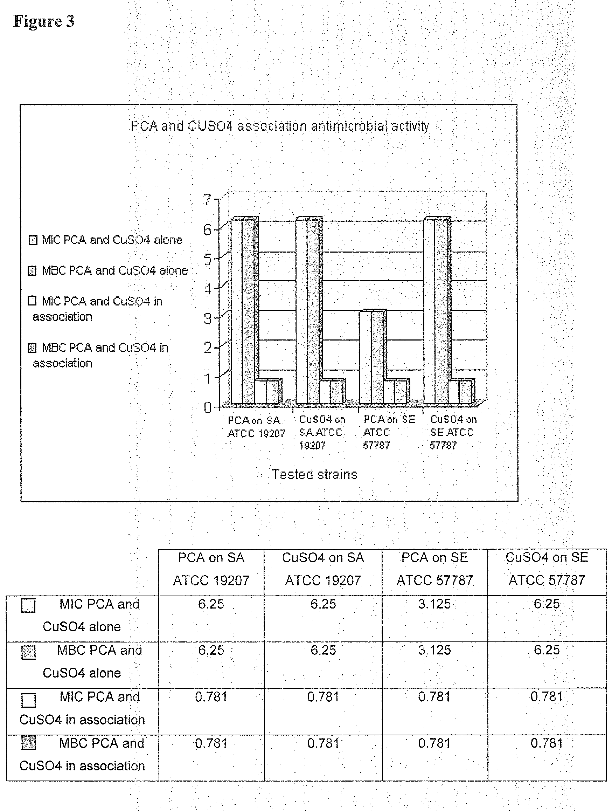

Figure 3: MIC and MBC of the association between PCA and CuSO₄ on *Staphylococcus aureus* (SA) and *Staphylococcus epidermidis* (SE)

### Description

Thus the disclosure provides a composition containing PCA and at least one metallic salt, preferably a copper, zinc or manganese salt, more preferably a copper salt, and at least one physiologically acceptable excipient.

The composition is preferably formulated in form of cream, gel, solution, suspension, stick, wet wipe or spray.

Said copper, zinc or manganese salt is preferably selected from among copper sulphate, zinc sulphate, manganese sulphate, zinc chloride, copper chloride, manganese chloride or mixtures thereof.

PCA is present in the composition according to the disclosure in non-salified form with said at least one metallic salt; i.e. PCA and said at least one metallic salt are present in the aforementioned composition as two separate components. This allows using - in the composition - an amount of said metallic salt varying from 0.0001 to 0.05% in weight, with respect to the total weight of the composition, and not molar amounts of said metallic salt with respect to the PCA (which would be required if PCA and metallic salt were inserted in the aforementioned composition mutually salified).

Actually, it was surprisingly discovered that said amounts which vary from 0.0001 to 0.05% in weight, with respect to the total weight of the composition, synergically amplify the antiviral and/or antibacterial activity of PCA without giving rise to toxic effects, for example irritation phenomena, due to the use of the metal at molar concentrations with respect to the PCA.

The expression "antiviral activity" according to the present invention, is used to indicate an activity against virus, in particular against influenza virus, such as influenza virus A, the influenza virus B and the influenza virus C, more specifically, it is used to indicate an activity against virus of the influenza A.

PCA is present in the composition according to the disclosure at an amount varying from 0.0001 to 5% in weight, with respect to the total weight of the composition.

Preferably, said metallic salt is present in the composition according to the invention at an amount varying from 0.0001 to 0.05% in weight, with respect to the total weight of the composition when formulated in a nasal spray, intimate detergent, paediatric intimate detergent or wet wipe sanitizer and at an amount varying from 0.001 to 0.05% in weight, with respect to the total weight of the composition when formulated in sanitizing spray, moisturizing cream or barrier cream.

Preferably, PCA is present in the composition at an amount varying from 0.0001 to 0.001% in weight, with respect to the total weight of the composition when formulated in nasal spray and at an amount varying from 0.001 to 5% in weight, with respect to the total weight of the composition when formulated in sanitizing spray, moisturizing cream, barrier cream, intimate cleansing wash, paediatric intimate cleansing wash and wet wipe sanitizer.

Furthermore, it was observed that the composition of the invention does not give rise to the system absorption of PCA and of said metallic salt: the action thereof remains exclusively topic.

Furthermore, as observed in the results outlined in the following examples, the composition of the invention is capable of performing a greater antibacterial and/or antiviral activity with respect to a composition containing PCA alone or the metallic salt alone, preferably copper sulphate.

The synergy, given by the composition of the invention, between PCA and metallic salt is thus proven.

According to an embodiment of the present invention, the composition is formulated as sanitizing spray, preferably as hand sanitizer spray.

The expression "sanitizing spray" according to the present invention is used to indicate a solution or suspension to be atomised, i.e. a spray indicated for the instantaneous, efficient and long-lasting disinfection of the skin (for example the hands) and of the mucosae, whose main properties are summarized in: disinfection, protection and/or prevention. Said spray allows obtaining a high hygiene level.

According to the disclosure, the hand sanitizer spray contains PCA, a metallic salt, preferably copper sulphate, and at least one physiologically acceptable excipient.

In said hand sanitizer spray, PCA is present in an amount preferably varying between 2 and 5% in weight, with respect to the total weight of the composition, in weight and said metallic salt, preferably copper sulphate, is present in an amount preferably varying between 0.001 and 0.05% in weight with respect to the total weight of the composition.According to a further embodiment of the present invention, the composition is formulated as a barrier cream.

The expression "barrier cream" according to the present invention is used to indicate a cream capable of protecting and/or shielding the skin against the attack of potentially aggressive external agents; said barrier cream preferably contains a high concentration of zinc oxide which performs a soothing and protective action on the skin.

According to the disclosure, the barrier cream contains PCA, a metallic salt, preferably copper sulphate, and at least one physiologically acceptable excipient.

In said barrier cream, PCA is present at an amount preferably varying between 0.5 and 4% in weight, with respect to the total weight of the composition, and said copper sulphate, is present in an amount preferably varying between 0.0005 and 0.05% in weight with respect to the total weight of the composition. According to a further embodiment of the present invention, the composition is formulated as moisturizing cream.

The expression "moisturizing cream" according to the present invention is used to indicate a cream capable of providing the required water to the skin and help it to retain the water; said moisturizing cream preferably contains a high concentration of moisturizing substances (for example, glycerine) and fats (for example, soy oil). According to the disclosure, the moisturizing cream contains PCA, a metallic salt, preferably copper sulphate, and at least one physiologically acceptable excipient.

In said moisturizing cream PCA is present in an amount preferably varying between 0.5 and 4% in weight, with respect to the total weight of the composition, and said copper sulphate, is present in an amount preferably varying between 0.0005 and 0.05% in weight with respect to the total weight of the composition.According to the disclosure, in said moisturizing cream the pH is corrected with pH regulating agents (for example, triethanolamine) to a value between 5 and 6.

According to a further embodiment of the present invention, the composition is formulated as intimate cleansing wash.

The expression "intimate cleansing wash" according to the present invention is used to indicate a specific detergent in form of gel for the cute and/or the mucosae of the ano-genital area, preferably characterized by a pH comprised between 3 and 5, more preferably about 4.

According to the disclosure, the intimate detergent contains PCA, a metallic salt, preferably copper sulphate, and at least one physiologically acceptable excipient.

In said intimate cleansing wash PCA is present in an amount preferably varying between 0.5 and 4% in weight, with respect to the total weight of the composition, and said copper sulphate, is present in an amount preferably varying between 0.0001 and 0.05% in weight with respect to the total weight of the composition.

According to a further embodiment of the present invention, the composition is formulated as paediatric intimate cleansing wash.

The expression "paediatric intimate cleansing wash" according to the present invention is used to indicate an intimate cleansing wash, as indicated above, intended for the "paediatric population", where the expression "paediatric population" is used to indicate the part of population between birth and eighteen years of age having a pH preferably comprised between 3 and 7, more preferably about 5.5.

According to the disclosure, the paediatric intimate cleansing wash contains PCA, a metallic salt, preferably copper sulphate, and at least one physiologically acceptable excipient.

In said paediatric intimate cleansing wash PCA is present in an amount preferably varying between 0.5 and 4% in weight, with respect to the total weight of the composition, and said copper sulphate, is present in an amount preferably varying between 0.0001 and 0.05% in weight, with respect to the total weight of the composition.

It should be observed that the intimate cleansing washes according to the invention do not modify - in any manner whatsoever - the lactobacillus charge usually present in the genital mucosae, where it performs the physiological action thereof maintaining the pH of the mucosal tissue at values not compatible with the growth of the pathogenic germs. According to a further embodiment of the present invention, the composition is formulated as a wet wipe sanitizer, preferably a wet wipe hand sanitizer.

The expression "wet wipe sanitizer" according to the present invention is used to indicate a tissue soaked with a substance having antiviral and/or antibacterial activity. According to the disclosure, the wipe contains PCA, a metallic salt, preferably copper sulphate, and at least one physiologically acceptable excipient.

In said wet wipe PCA is present in an amount preferably varying between 0.005 and 5% in weight with respect to the total weight of the composition and said copper sulphate, is present in an amount preferably varying between 0.0001 and 0.001% in weight, with respect to the total weight of the composition. According to a further description, the composition is formulated as nasal spray.

The expression "nasal spray" according to the present invention is used to indicate a solution and/or suspension to be atomised on the nasal mucosa, optionally containing a saline solution, thermal water or mixtures thereof. Said solution and/or suspension may be isotonic, hypotonic or hypertonic with respect to body fluids.

In said nasal spray PCA is present in an amount preferably varying between 0.0001 and 0.001% in weight, with respect to the total weight of the composition, more preferably in an amount equivalent to about 0.0005% in weight and said metallic salt, preferably copper sulphate, is present in an amount preferably varying between 0.0001 and 0.001% in weight with respect to the total weight of the composition, more preferably in an amount equivalent to about 0.0005% in weight.

In a further embodiment of the present invention, the composition contains PCA and copper sulphate in association with a further component.

Said further component is preferably glutaric acid, saline solution, thermal water or mixtures thereof.

The composition according to the invention has the advantage of containing neither ethyl alcohol nor paraben preservatives, contrary to the compositions currently available in the market.

Ethyl alcohol is usually used in the compositions with antiviral and/or antibacterial activity for skin application with the aim of reducing the microbial flora. The use thereof, especially long-term, however causes adverse effects on the skin such as dehydration and reddening.

Parabens, which are the most commonly used preservatives in the cosmetic industry, often cause allergies. The fact that they are not present in the composition of the invention allows optimizing the skin tolerability of the composition.

In another disclosure, the composition also reveals an antihistamine activity, in particular on insect stings.

Furthermore, PCA in the composition of the invention, besides performing an antiviral and/or antibacterial action on the skin, also ensures a moisturizing and emollient action. A further advantage of the present invention lies in the fact that the composition according to the invention performs an antiviral and/or antibacterial action which lasts up to about 4 hours, contrary to the compositions currently available on the market which perform an antiviral action which lasts only for about 30 minutes.

### Experimental part

### Examples

Some formulation examples are indicated in the following tables.

### Example 1

**Sanitizing spray**

| **COMPONENTS** | **% of weight** |
|---|---|
| PCA | 4.000 |
| Copper sulphate | 0.002 |
| Sodium hydroxide | 3.100 |
| perfume | 0.931 |
| sodium chloride | 0.900 |
| Propylene glycol | 0.623 |
| Imidazolidinyl urea | 0.300 |
| chlorphenesin | 0.070 |
| limonene | 0.051 |
| citral | 0.017 |
| methylisothiazolinone | 0.007 |
| linalool | 0.0002 |
| geraniol | 0.0001 |
| Water qs to 100 | |

### Example 2

**Barrier cream**

| **PRODUCT** | **% weight** |
|---|---|
| PCA | 1 |
| Copper sulphate | 0.001 |
| Zinc oxide | 8 |
| Glycerin | 10 |
| Soy oil | 7 |
| Sorbitol a 70% | 5 |
| Cetiol v | 3 |
| Cetostearyl alcohol | 3 |
| Glyceryl myristate | 3 |
| Amphisol (DEA-cetyl phosphate) | 2 |
| Titanium oxide | 2 |
| Xanthan gum | 0.8 |
| Seba cream essence | 0.8 |
| Panthenol | 0.5 |
| Benzoic acid | 0.4 |
| Sorbic acid | 0.4 |
| Vitamin E | 0.4 |
| Lecifarma Chamomile liquid extract | 0.05 |
| Water qs to 100 | |

### Example 3

**Moisturizing cream**

| **PRODUCT** | **% weight** |
|---|---|
| PCA | 1 |
| Copper sulphate | 0.001 |
| Sorbitol al 70% | 10 |
| Soy oil | 6 |
| Glycerin | 5 |
| Cetiol v | 2 |
| Cetyl alcohol | 2 |
| Isopropyl myristate | 2 |
| Cremophor A25 (BASF) 1 1 | 1 |
| Cremophor A6 (BASF) 1 1 | 1 |
| Carbopol 940 | 0.8 |
| Sebacream essence | 0.8 |
| Panthenol | 0.5 |
| Silicone oil | 0.5 |
| Benzoic acid | 0.4 |
| Sorbic acid | 0.4 |
| Vitamin E | 0.4 |
| Chamomile liquid extract | 0.05 |
| Triethanolamine | q.b. |
| Water qs to 100 | |

### Example 4

**Paediatric intimate cleansing wash**

| **COMPONENTS** | **% weight** |
|---|---|
| Sodium dilaureth-7 citrate | 3.00 |
| Hydroxyethylcellulose | 1.50 |
| Peg 20 glyceryl ricinoleate | 1.05 |
| PCA | 1.00 |
| Trideceth-9 | 0.81 |
| Propylene glycol | 0.62 |
| Potassium cocoyl hydrolyzed soy protein | 0.60 |
| Peg-40 hydrogenated castor oil | 0.51 |
| Perfume | 0.50 |
| Disodium laureth sulfosuccinate | 0.50 |
| Ricinoleamide dea | 0.45 |
| Sodium lauryl sulfoacetate | 0.40 |
| Sodium hydroxide | 0.31 |
| Disodium edta | 0.10 |
| Chlorphenesin | 0.07 |
| Polysorbate 20 | 0.06 |
| Methylisothiazolinone | 0.0066 |
| Water qs to 100 | |

### In vitro test

In vitro tests were conducted to evaluate the safety and efficacy of the various embodiments of the invention.

### a) "Humana Patch test" on sensitive skin

The product was applied on the skin by means of a patch containing the product to be tested.

The evaluation of the adverse effects following the application of the product was conducted on 25 healthy volunteers.

The skin reactions were evaluated clinically after 15 minutes, 1 hour, 24 hours, 48 hours from the time of application.

No irritation, oedematous effect and no erythema was observed at various times, thus allowing classifying the product as non-irritating according to the Draize method.

### b) Antibacterial activity

The antibacterial activity was studied through an in vitro quantitative method regarding two different bacterial strains (*Staphylococcus aureus* (SA) ATCC 19207 and *Staphylococcus epidermidis* (SE) ATCC 57787) using various compositions of the invention containing PCA alone, copper sulphate alone or PCA and copper sulphate combined at different concentrations.

The bacterial strains were acquired from the University of Goteborg, Sweden (CCUG, Culture Collection, University of Goteborg).

The consumption material, the plates and the media were acquired from Biomerieux, Florence.

The bacterial strains were seeded in solid blood agar medium and kept in incubation at 37 °C for 24 hours; then, a bacterial suspension in Brucella Bovine Serum broth, equivalent to 0.5 of the McFarland turbidimetric standard (solution of 0.05 mL of BaCl₂ at 1% and 9.95 mL of H₂SO₄ at 1%) approximately corresponding to a bacterial suspension of 1 x 10⁷ UFC/mL was prepared for each strain. The suspensions thus obtained were diluted 100 times to obtain a 1 x 10⁵ UFC/mL bacterial concentration. For each bacterial strain there were joined two 96 wells (8 x 12) multiwell plates so as to have 16 x 12 available wells.

In the well 1A and 1B, the first on the top left in the first plate and the corresponding underlying one, there were arranged 200 µL of the samples solubilised in broth with concentration of pyrrolidone carboxylic acid (PCA) at 100 mg/mL and copper sulphate pentahydrate (CuSO₄) 25 mg/mL. In the wells from 2A to 12A and from 2B to 12B there were arranged 100 µL of broth and 100 µL were transferred from time to time from the well on the left so as to obtain serial dilutions and 12 concentrations for each sample tested separately.

In the rest of the column 1 of the two plates there were arranged 200 µL of CuSO₄ 25 mg/mL which were serially diluted horizontally from well 1 to 12 of each row. In the entire row C, in which CuSO₄ was already present at different concentrations, there were then introduced 100 µL of PCA 100 mg/mL; the dilutions of the PCA were conducted vertically so as to obtain all possible combinations concentration of the mixture of the samples.

The well 1C contains PCA 50 mg/mL and CuSO₄ 12.5 mg/mL; the well 2C PCA 50 mg/mL and CuSO₄ 6.25 mg/mL; the well 3C PCA 50 mg/mL and CuSO₄ 3.125 mg/mL and so on and so forth along the row. The well ID instead contains PCA 25 mg/mL and CuSO₄ 12.5 mg/mL; the well 1E PCA 12.5 mg/mL and CuSO₄ 12.5 mg/mL and so on and so forth along the column. The fact that the dilutions of PCA are conducted vertical and those of CuSO₄ are conducted horizontal allows knowing the concentration of the constituents in each well of the plates.

The last row contains only 100 µL of broth culture as reference.

10 µL of the bacterial suspensions were added in each well.

The inhibition scheme on the multiwell is represented below:
*CuSO₄ 100 mM = 25 mg*/*mL*
*CuSO₄ 50 mM = 12.5 mg*/*mL*
*CuSO₄ 25 mM = 6.25 mg*/*mL*
*CuSO₄ 12.5 mM = 3.125 mg*/*mL*
*CuSO₄ 6.25 mM = 1.563 mg*/*mL*
*CuSO₄ 3.125 mM = 0.781 mg*/*mL*

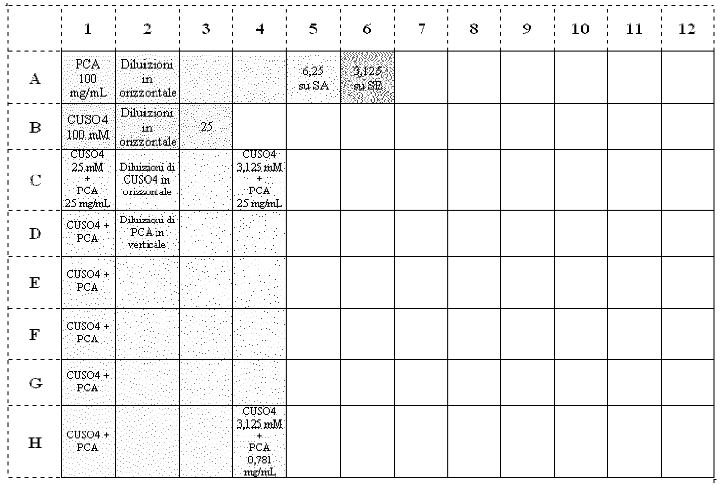

The dilutions continue in a second plate (starting from the concentration of PCA 0.391 mg/mL) but inhibition is only observed in the column 1 due to the action of CuSO₄ (confirmed at 25 mM = 6.25 mg/mL).

The plates were incubated for 24 hours at 37 °C and then 3 µL of each dilution were transferred on blood agar plates which were incubated for 1 day.

The lower concentration, whose subculture on plate revealed the absence of growth, was considered the minimum bactericidal concentration (MBC) while the lower concentration in the wells that did not reveal bacterial growth (easily detectable due to a visible white colonisation on the bottom of the well) was considered the minimum inhibitory concentration (MIC).

The tests were conducted in triples with independent experiments.

PCA revealed an MIC on *Staphylococcus aureus* (SA) equivalent to 6.25 mg/mL. The subculture on Petri dish confirmed that the MBC corresponds to the MIC.

On *Staphylococcus epidermidis* (SE) PCA has MIC and MBC equivalent to 3.125 mg/mL.

Also CuSO₄ revealed MIC and MBC matching and, contrary to PCA, it did not reveal different actions on different strains: on SA and SE, MIC and MBC are equivalent to 6.25 mg/mL.

The most important results derive from the synergy of action of the samples: on SA, MIC and MBC of the mixture of constituents is PCA 0.781 mg/mL / CuSO₄ 0.781 mg/mL, i.e. 8 times better than PCA alone and 8 times better than CuSO₄ alone.

On SE the results of synergy between the constituents were the same of the experiment conducted on SA: MIC and MBC equivalent to PCA 0.781 mg/mL / CuSO₄ 0.781 mg/mL.

The triplicate of the experiments did not vary the result thus validating the experiment and the obtained data.

### c) Antiviral activity

The antiviral activity was conducted *in vitro* using Rhinovirus type 37 produced in HeLa cells (American Type Culture Collection, Mass.).

In particular, from the obtained experimental results, there can be observed a reduction of the viral count (expressed in log of the concentration) when the composition according to the invention containing PCA and copper sulphate is used with respect to the other compositions in which PCA is used alone or copper sulphate alone.

## Claims

1. Composition containing pyrrolidone carboxylic acid, copper sulphate and at least one physiologically acceptable excipient, **characterised in that** it is formulated as a sanitizing spray wherein pyrrolidone carboxylic acid is present in an amount varying between 2 and 5% in weight with respect to the total weight of the composition, and said copper sulphate is present in an amount varying between 0.001 and 0.05% in weight with respect to the total weight of the composition.

2. Composition containing pyrrolidone carboxylic acid, copper sulphate and at least one physiologically acceptable excipient, **characterised in that** it is formulated as a barrier cream wherein pyrrolidone carboxylic acid is present in an amount varying between 0.5 and 4% in weight with respect to the total weight of the composition, and said copper sulphate is present in an amount varying between 0.0005 and 0.05% in weight, with respect to the total weight of the composition.

3. Composition containing pyrrolidone carboxylic acid, copper sulphate and at least one physiologically acceptable excipient, **characterised in that** it is formulated as a moisturizing cream wherein pyrrolidone carboxylic acid is present in an amount varying between 0.5 and 4% in weight with respect to the total weight of the composition, and said copper sulphate is present in an amount varying between 0.0005 and 0.05% in weight with respect to the total weight of the composition.

4. Composition containing pyrrolidone carboxylic acid, copper sulphate and at least one physiologically acceptable excipient, **characterised in that** it is formulated as an intimate cleansing wash wherein pyrrolidone carboxylic acid is present in an amount varying between 0.5 and 4% in weight with respect to the total weight of the composition, and said copper sulphate is present in an amount varying between 0.0001 and 0.05% in weight with respect to the total weight of the composition.

5. Composition containing pyrrolidone carboxylic acid, copper sulphate and at least one physiologically acceptable excipient, **characterised in that** it is formulated as a paediatric intimate cleansing wash wherein pyrrolidone carboxylic acid is present in an amount varying between 0.5 and 4% in weight with respect to the total weight of the composition, and said copper sulphate is present in an amount varying between 0.0001 and 0.05% in weight with respect to the total weight of the composition.

6. Composition containing pyrrolidone carboxylic acid, copper sulphate and at least one physiologically acceptable excipient, **characterised in that** it is formulated as a wet wipe sanitizer wherein pyrrolidone carboxylic acid is present in an amount varying between 0.005 and 5% in weight with respect to the total weight of the composition, and said copper sulphate is present in an amount preferably varying between 0.0001 and 0.001% in weight, with respect to the total weight of the composition.

7. Composition according to any one of the preceding claims, **characterised in that** it contains a further component.

8. Composition according to claim 7, **characterised in that** said further component is glutaric acid, saline solution, thermal water or mixtures thereof.

## Patentansprüche

1. Zusammensetzung umfassend Pyrrolidonkarboxylsäure, Kupfersulfat und mindestens einen physiologisch akzeptierbaren Hilfsstoff, **dadurch gekennzeichnet, dass** sie formuliert ist als desinfizierendes Spray, wobei Pyrrolidonkarboxylsäure vorliegt in einer Menge, welche variiert zwischen 2 und 5 Gewichts-% relativ zu dem Gesamtgewicht der Zusammensetzung, und wobei Kupfersulfat vorliegt in einer Menge variierend zwischen 0,001 und 0,05 Gewichts-% relativ zu dem Gesamtgewicht der Zusammensetzung.

2. Zusammensetzung beinhaltend Pyrrolidonkarboxylsäure, Kupfersulfat und mindestens einen physiologisch akzeptierbaren Hilfsstoff, **dadurch gekennzeichnet, dass** sie formuliert ist als eine Schutzcreme, wobei Pyrrolidonkarboxylsäure in einer Menge vorliegt, welche variiert zwischen 0,5 und 4 Gewichts-% relativ zu dem Gesamtgewicht der Zusammensetzung, und wobei Kupfersulfat vorliegt in einer Menge, welche variiert zwischen 0,0005 und 0,05 Gewichts-% relativ zu dem Gesamtgewicht der Zusammensetzung.

3. Zusammensetzung beinhaltend Pyrrolidonkarboxylsäure, Kupfersulfat und mindestens einen physiologisch akzeptierbaren Hilfsstoff, **dadurch gekennzeichnet, dass** sie formuliert ist als eine Feuchtigkeitscreme, wobei Pyrrolidonkarboxylsäure vorliegt in einer Menge, welche variiert zwischen 0,5 und 4 Gewichts-% relativ zu dem Gesamtgewicht der Zusammensetzung, und wobei Kupfersulfat vorliegt in einer Menge, welche variiert zwischen 0,0005 und 0,05 Gewichts-% relativ zu dem Gesamtgewicht der Zusammensetzung.

4. Zusammensetzung beinhaltend Pyrrolidonkarboxylsäure, Kupfersulfat und mindestens einen physiologisch akzeptierbaren Hilfsstoff, **dadurch gekennzeichnet, dass** sie formuliert ist als ein Intimreinigungsmittel, wobei Pyrrolidonkarboxylsäure vorliegt in einer Menge, welche variiert zwischen 0,5 und 4 Gewichts-% relativ zu dem Gesamtgewicht der Zusammensetzung, und wobei Kupfersulfat vorliegt in einer Menge, welche variiert zwischen 0,0001 und 0,05 Gewichts-% relativ zu dem Gesamtgewicht der Zusammensetzung.

5. Zusammensetzung beinhaltend Pyrrolidonkarboxylsäure, Kupfersulfat und mindestens einen physiologisch akzeptierbaren Hilfsstoff, **dadurch gekennzeichnet, dass** sie formuliert ist als pädiatrisches Intimreinigungsmittel, wobei Pyrrolidonkarboxylsäure vorliegt in einer Menge, welche variiert zwischen 0,5 und 4 Gewichts-% relativ zu dem Gesamtgewicht der Zusammensetzung und wobei Kupfersulfat vorliegt in einer Menge, welche variiert zwischen 0,0001 und 0,05 Gewichts-% relativ zu dem Gesamtgewicht der Zusammensetzung.

6. Zusammensetzung beinhaltend Pyrrolidonkarboxylsäure, Kupfersulfat und mindestens einen physiologisch akzeptierbaren Hilfsstoff, **dadurch gekennzeichnet, dass** sie formuliert ist als Feuchttuchreiniger, wobei Pyrrolidonkarboxylsäure vorliegt in einer Menge, welche variiert zwischen 0,005 und 5 Gewichts-% relativ zu dem Gesamtgewicht der Zusammensetzung, und wobei Kupfersulfat vorliegt in einer Menge, welche vorzugsweise variiert zwischen 0,0001 und 0,001 Gewichts-% relativ zu dem Gesamtgewicht der Zusammensetzung.

7. Zusammensetzung gemäß einem der vorstehenden Ansprüche, **dadurch gekennzeichnet, dass** sie eine weitere Komponente beinhaltet.

8. Zusammensetzung gemäß Anspruch 7, **dadurch gekennzeichnet, dass** die weitere Komponente Glutarsäure, eine Salzlösung, Thermalwasser oder Mischungen daraus ist.

## Revendications

1. Composition contenant de l'acide pyrrolidone-carboxylique, du sulfate de cuivre et au moins un excipient physiologiquement acceptable, **caractérisée en ce qu'**elle est formulée sous la forme d'un spray désinfectant, dans laquelle l'acide pyrrolidone-carboxylique est présent en une quantité variant entre 2 et 5 % en poids par rapport au poids total de la composition, et ledit sulfate de cuivre est présent en une quantité variant entre 0,001 et 0,05 % en poids par rapport au poids total de la composition.

2. Composition contenant de l'acide pyrrolidone-carboxylique, du sulfate de cuivre et au moins un excipient physiologiquement acceptable, **caractérisée en ce qu'**elle est formulée sous la forme d'une crème formant barrière, dans laquelle l'acide pyrrolidone-carboxylique est présent en une quantité variant entre 0,5 et 4 % en poids par rapport au poids total de la composition, et ledit sulfate de cuivre est présent en une quantité variant entre 0,0005 et 0,05 % en poids par rapport au poids total de la composition.

3. Composition contenant de l'acide pyrrolidone-carboxylique, du sulfate de cuivre et au moins un excipient physiologiquement acceptable, **caractérisée en ce qu'**elle est formulée sous la forme d'une crème hydratante, dans laquelle l'acide pyrrolidone-carboxylique est présent en une quantité variant entre 0,5 et 4 % en poids par rapport au poids total de la composition, et ledit sulfate de cuivre est présent en une quantité variant entre 0,0005 et 0,05 % en poids par rapport au poids total de la composition.

4. Composition contenant de l'acide pyrrolidone-carboxylique, du sulfate de cuivre et au moins un excipient physiologiquement acceptable, **caractérisée en ce qu'**elle est formulée sous la forme d'un lavage nettoyant à usage intime, dans laquelle l'acide pyrrolidone-carboxylique est présent en une quantité variant entre 0,5 et 4 % en poids par rapport au poids total de la composition, et ledit sulfate de cuivre est présent en une quantité variant entre 0,0001 et 0,05 % en poids par rapport au poids total de la composition.

5. Composition contenant de l'acide pyrrolidone-carboxylique, du sulfate de cuivre et au moins un excipient physiologiquement acceptable, **caractérisée en ce qu'**elle est formulée sous la forme d'un lavage nettoyant à usage intime pédiatrique, dans laquelle l'acide pyrrolidone-carboxylique est présent en une quantité variant entre 0,5 et 4 % en poids par rapport au poids total de la composition, et ledit sulfate de cuivre est présent en une quantité variant entre 0,0001 et 0,05 % en poids par rapport au poids total de la composition.

6. Composition contenant de l'acide pyrrolidone-carboxylique, du sulfate de cuivre et au moins un excipient physiologiquement acceptable, **caractérisée en ce qu'**elle est formulée sous la forme d'une lingette désinfectante humide, dans laquelle l'acide pyrrolidone-carboxylique est présent en une quantité variant entre 0,005 et 5 % en poids par rapport au poids total de la composition, et ledit sulfate de cuivre est présent en une quantité variant entre 0,0001 et 0,001 % en poids par rapport au poids total de la composition.

7. Composition selon l'une quelconque des revendications précédentes, **caractérisée en ce qu'**elle contient un autre composant.

8. Composition selon la revendication 7, **caractérisée en ce que** ledit autre composant est l'acide glutarique, une solution salée, de l'eau thermale ou des mélanges de ceux-ci.
